# EUROPEAN PATENT APPLICATION

(11) **EP 3 124 949 A1**
(43) Date of publication of application: **01.02.2017**
(21) Application number: 15769731.9
(22) Date of filing: 16.03.2015
(51) Int. Cl.: G01M 13/04

(54) **TESTING DEVICE FOR THIN-WALLED LARGE-SIZED BEARING**

(30) Priority: 22.03.2014 JP 2014059350
(71) Applicant: NTN Corporation, Osaka-shi, Osaka 550-0003 (JP)
(72) Inventor: KARASAWA, Miki, Kuwana-shi, Mie 511-8678 (JP); KOHORI, Ken, Kuwana-shi, Mie 511-8678 (JP)
(74) Representative: Wagner, Kilian
(86) International application number: PCT/JP2015/057769
(87) International publication number: WO 2015/146687

(57) **Abstract**

A testing device includes: a face board allowing a thin-walled large-sized bearing as a test target to be installed thereon; a face board support mechanism supporting the face board such that the face board can be tilted about a tilting central shaft; an angle change drive device for changing a tilt angle of the face board; a bearing rotation motor for rotating an inner ring of the bearing; and a weight for generating a moment load to the bearing. The face board support mechanism and the angle change drive device are capable of changing the orientation of the face board in a range from a horizontal orientation through a vertical orientation to a tilted orientation. The face board is provided with a dedicated frame to be fitted to an outer circumference of the bearing and attached in a fitted manner to the face board.

## Description

### CROSS REFERENCE TO THE RELATED APPLICATION

This application is based on and claims Convention priority to Japanese patent application No. 2014-059350, filed March 22, 2014, the entire disclosure of which is herein incorporated by reference as a part of this application.

### BACKGROUND OF THE INVENTION

### (Field of the Invention)

The present invention relates to a testing device for conducting various tests of a thin-walled large-sized bearing used in, for example, a CT scanner device which is a medical examination device.

### (Description of Related Art)

A CT scanner device (for example, Fig. 13 in Patent Document 1) has, as an examination unit, a circular rotating mount (gantry) which allows a cradle on which an examination subject such as a patient is laid to enter thereto and exit therefrom. The rotating mount is rotatably supported via a bearing on a fixing unit on the outer circumferential side, and is provided with an X-ray tube, an X-ray detector, and the like for capturing an image. The bearing used for supporting the rotating mount is a thin-walled large-sized bearing with an outer diameter of about 600 to 1300 mm, and a load of about 1 t from mounted components such as the X-ray tube is applied to an inner ring of the bearing. Depending on a part to be imaged, such as an eyeball, the angle of the imaging needs to be changed, and therefore some CT scanner devices are configured such that the tilt angle of the rotating mount can be changed. In such CT scanner devices, the load applied to the bearing varies depending on the tilt angle of the rotating mount.

Generally, the bearing for supporting the rotating mount of the CT scanner device is required to have the following performances.
(1) Silence. This prevents an organ from being contracted due to patient's tension, and gives a feeling of relief to the patient, to keep the organ acting normally.
(2) Low vibration. The lower the vibration is, the higher the quality of a captured image is.
(3) High speed. Shortening a time needed for imaging reduces the X-ray exposed dose.

In order to assure the above performances of the bearing for supporting the rotating mount, it is necessary to conduct a performance evaluation test before the bearing is incorporated into the CT scanner device. An example of such testing devices is disclosed in Patent Document 2. This testing device holds the bearing such that the bearing can be tilted at any angle, by a bearing holding mechanism, and allows an inner ring of the held bearing to be rotated at any rotation speed, thus enabling a performance evaluation test of the bearing under various conditions.

### [Related Document]

### [Patent Document]

[Patent Document 1] JP Laid-open Patent Publication No. 2012-82844
[Patent Document 2] JP Laid-open Patent Publication No. 2005-315681

### SUMMARY OF THE INVENTION

Patent Document 2 describes that, since the baring can be tilted at any angle, the testing device is capable to make "the attachment orientation of the bearing be the same as that of, for example, a rolling bearing used for supporting a CT scanner gantry head". However, the specification of Patent Document 2 does not disclose that the bearing can be held horizontally. In addition, since the testing device is such a type that the bearing is fixed by being fitted into a depressed portion of a housing, the bearing can be fixed to the housing even when the bearing is in a vertical orientation. Thus, it is considered that, in this testing device, the bearing is attached to the bearing holding mechanism in a state in which the bearing is set in a vertical orientation or in a tilted orientation. Since the bearing used in the CT scanner device is large and heavy, it is not easy to attach the bearing to the bearing holding mechanism while a person supports the bearing in an appropriate orientation.

An object of the present invention is to provide a testing device for a thin-walled large-sized bearing, that allows the thin-walled large-sized bearing as a test target to be easily and accurately installed, allows a performance evaluation test to be conducted with the bearing tilted in any orientation, and further allows a performance evaluation test to be conducted when a moment load is applied to the bearing and a performance evaluation test to be conducted when an eccentric load is applied to the bearing.

A testing device for a thin-walled large-sized bearing of the present invention includes: a face board configured to allow the thin-walled large-sized bearing as a test target to be installed thereon; a face board support mechanism configured to support the face board such that the face board can be tilted about a tilting central shaft of the face board aligned with or parallel to a diameter line of the bearing installed on the face board, with the tilting central shaft being horizontally supported; an angle change drive device configured to change a tilt angle of the face board; a bearing rotation motor installed on the face board and configured to rotate an inner ring of the bearing; and a weight detachably attached to the inner ring of the bearing, to generate a moment load to the bearing. The face board support mechanism and the angle change drive device cooperatively support the face board so as to allow change in an orientation of the face board in a range from a horizontal orientation to a vertical orientation through a tilted orientation. The face board is provided with a dedicated frame fitted to an outer circumference of the bearing and attached in a fitted manner to a frame installation hole formed in the face board. The moment load is a moment given to the bearing as the test target by a load acting thereon in a direction perpendicular to the central axis of the bearing at a position separated in the axial direction from the bearing as the test target.

The "thin-walled large-sized bearing" as the test target in the present invention refers to a bearing in which a difference between an inner diameter and an outer diameter thereof with respect to the inner diameter is greater than that of a general bearing, and the inner diameter is great, for example, a bearing in which a value of (outer diameter - inner diameter) / (inner diameter) is 0.3 or smaller and the inner diameter is 600 mm or greater.

According to the above configuration, the face board is set in the horizontal orientation by the face board support mechanism and the angle change drive device, and then the dedicated frame fitted to the outer circumference of the bearing as the test target is attached in a fitted manner to the frame installation hole of the face board set in the horizontal state, whereby the bearing as the test target is installed on the face board. Since the face board is horizontal, it is only necessary to lower the dedicated frame or the bearing set in the horizontal orientation from above onto the face board in installation of the bearing. It is not necessary to support the dedicated frame or the bearing in a proper orientation by a person. Therefore, even if the bearing as the test target is the thin-walled large-sized bearing with a great weight, the bearing as the test target can be easily installed on the face board. Since the dedicated frame is fitted to the frame installation hole of the face board, and the outer circumference of the bearing as the test target is fitted to the inner circumferential surface of the dedicated frame, the positions of the dedicated frame and the bearing as the test target are prevented from being displaced relative to the upper surface of the face board. Therefore, the bearing as the test target can be accurately installed at the center of the face board.

After the bearing is installed on the face board, the face board is set in the vertical orientation or the tilted orientation, and the inner ring of the bearing installed on the face board is rotated by the bearing rotation motor, whereby a performance evaluation test of the bearing is conducted. Since the angle of the face board can be changed to any tilt angle or a predetermined tilt angle, the performance evaluation test of the bearing can be conducted in a state close to a usage state. By applying a moment load to the bearing as the test target by the weights, it becomes possible to conduct a performance evaluation test in a state in which a moment is applied to the bearing. Further, since the dedicated frame is fitted to the outer circumference of the bearing and the dedicated frame is fitted to the frame installation hole of the face board, even if an eccentric load that is eccentric with respect to the central axis of the bearing is applied to the bearing as the test target by the weights, the load can be received by the face board via the dedicated frame. Therefore, it is also possible to conduct a performance evaluation test when an eccentric load is applied to the bearing as the test target. In addition, owing to the configuration in which the bearing is installed on the face board via the dedicated frame, it is possible to adapt to plural types of bearings as described later, unlike the case where the bearing is directly installed on the face board.

In one embodiment of the present invention, the dedicated frame may have: an inner circumferential surface to be fitted to the outer circumference of the bearing; a receiving surface which protrudes radially inward from one end in an axial direction of the inner circumferential surface to receive a side surface of the bearing; an outer circumferential surface to be fitted to the frame installation hole of the face board; and a flange portion which protrudes from one end in the axial direction of the outer circumferential surface and is mounted on the face board.

Since the dedicated frame has the receiving surface for receiving the side surface of the bearing, an axial-direction load from the bearing as the test target can be received by the dedicated frame. In addition, since the dedicated frame has the flange portion to be mounted on the face board, an axial-direction load from the dedicated frame can be received by the face board. Therefore, it is possible to stably support the bearing as the test target.

In one embodiment of the present invention, as the dedicated frame, plural types of dedicated frames may be provided which can be installed in an exchangeable manner to the face board and which respectively allow bearings having different outer diameters to be fitted thereto.

In this case, it is possible to adapt to plural types of bearings having different outer diameters by exchanging the dedicated frame in accordance with the outer diameter of the bearing as the test target.

In one embodiment of the present invention, a face board fixing mechanism configured to fix the face board to the face board support mechanism at any tilt angle or a predetermined tilt angle may be provided separately from the angle change drive device.

By providing the face board fixing mechanism, the tilt angle of the face board can be reliably prevented from being unintentionally changed during a test or the like, thus ensuring security.

Thus, the testing device for the thin-walled large-sized bearing of the present invention is suitable for conducting a performance evaluation test of a bearing for supporting a rotating mount of a CT scanner device.

Any combination of at least two constructions, disclosed in the appended claims and/or the specification and/or the accompanying drawings should be construed as included within the scope of the present invention. In particular, any combination of two or more of the appended claims should be equally construed as included within the scope of the present invention.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a front view of a testing device for a thin-walled large-sized bearing according to an embodiment of the present invention;
Fig. 2 is a side view of the testing device for the thin-walled large-sized bearing;
Fig. 3A is a side view schematically showing a vertical orientation state of the testing device for the thin-walled large-sized bearing;
Fig. 3B is a side view schematically showing a tilted orientation state of the testing device for the thin-walled large-sized bearing;
Fig. 3C is a side view schematically showing a horizontal orientation state of the testing device for the thin-walled large-sized bearing;
Fig. 4 is a side view of a face board fixing mechanism of the testing device for the thin-walled large-sized bearing;
Fig. 5 is a view including a perspective view of the face board fixing mechanism and a partial enlarged view thereof;
Fig. 6 is a broken side view of a part of the testing device for the thin-walled large-sized bearing;
Fig. 7A is a sectional view showing an example of a dedicated frame used for the testing device for the thin-walled large-sized bearing;
Fig. 7B is a sectional view showing another example of the dedicated frame used for the testing device for the thin-walled large-sized bearing; and
Fig. 8 is a sectional view of an example of a CT scanner device.

### DESCRIPTION OF EMBODIMENTS

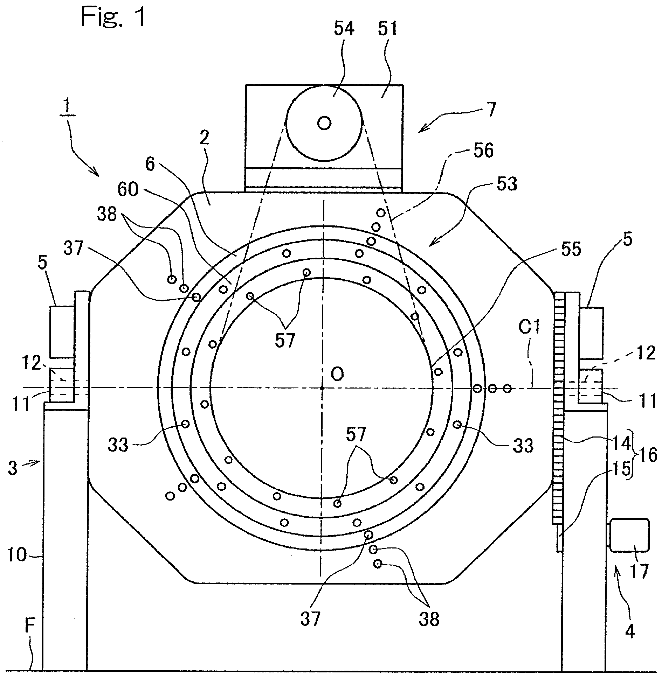

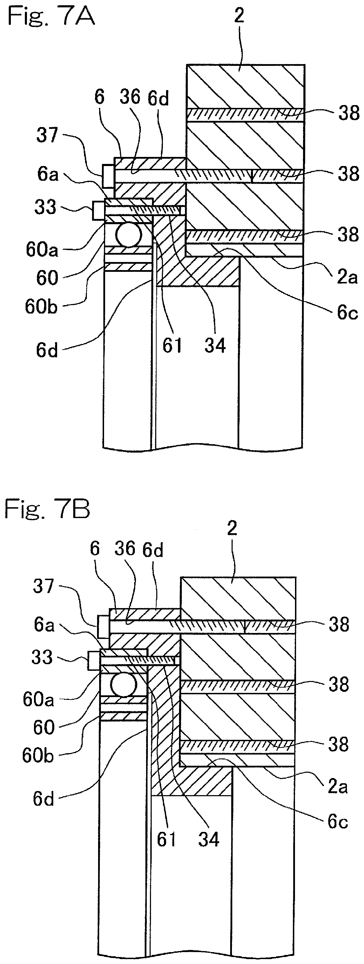

An embodiment of a testing device for a thin-walled large-sized bearing according to the present invention will be described with reference to Fig. 1 to Figs. 7A, 7B.

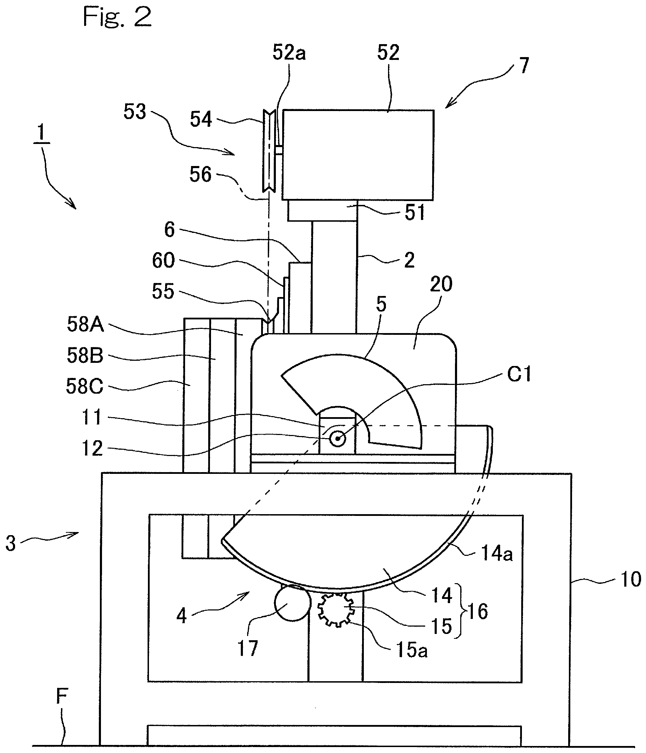

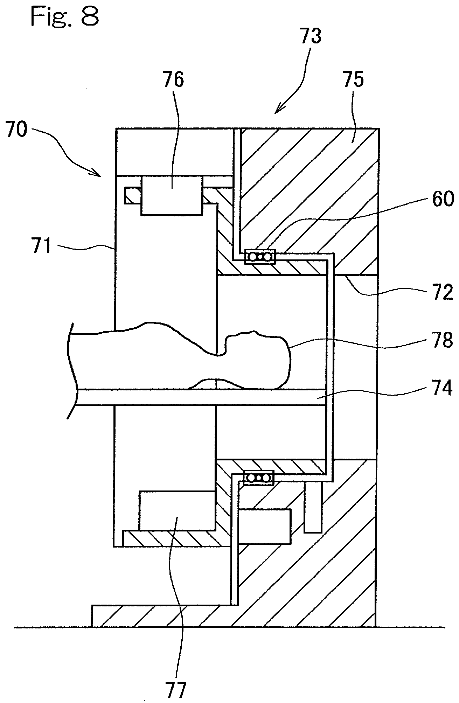

Fig. 1 is a front view of the testing device for the thin-walled large-sized bearing, and Fig. 2 is a side view thereof. The testing device 1 for the thin-walled large-sized bearing includes a face board 2, a face board support mechanism 3, an angle change drive device 4, a face board fixing mechanism 5, a dedicated frame 6 for bearing installation, a bearing rotating mechanism 7, and weights 58A, 58B, and 58C for generating a moment load to a bearing 60 which is a test target. The bearing 60 as the test target to be subjected to a performance evaluation test by the testing device 1 is the thin-walled large-sized bearing used for supporting, for example, a rotating mount 71 such as a gantry of a CT scanner device 70 shown in Fig. 8. The thin-walled large-sized bearing is formed as, for example, a rolling bearing such as a deep groove ball bearing, an angular contact ball bearing, a 4-point contact ball bearing, a cylindrical roller bearing, or a tapered roller bearing. The CT scanner device 70 will be described later.

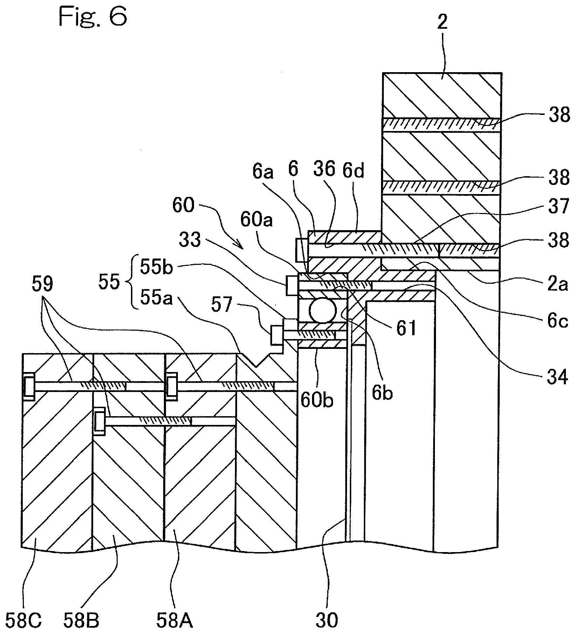

In Figs. 1 and 2, the face board 2 is used for installing thereon the bearing 60 as the test target, which is the thin-walled large-sized bearing. In this example, the face board 2 is in the form of a ring-like plate body having an octagonal outer shape and having a frame installation hole 2a (Fig. 6) at the center thereof.

The face board support mechanism 3 includes a frame 10 placed on a floor surface F and a pair of right and left tilting support bearing mechanisms 11 provided at an upper end of the frame 10. The face board support mechanism 3 rotatably supports a pair of tilting central shafts 12 protruding from right and left side surfaces of the face board 2, by the pair of right and left tilting support bearing mechanisms 11, respectively. A central axis C1 of the tilting central shaft 12 horizontally extends along the right-left direction, and is aligned with a diameter line of the bearing 60 as the test target installed on the face board 2. Instead of aligned with the diameter line of the bearing 60 as the test target, the central axis C1 of the tilting central shaft 12 may be parallel to the diameter line of the bearing 60 as the test target. By the face board support mechanism 3, the face board 2 is supported such that the face board 2 can be tilted about the central axis C1 of the tilting central shaft 12.

The angle change drive device 4 is a device for changing a tilt angle of the face board 2 about the central axis C1 of the tilting central shaft 12. The angle change drive device 4 includes a rotation transmission mechanism 16 including a to-be-rotated element 14 fixed on one (in the example shown in the drawings, right one) of the right and left side surfaces of the face board 2; and a rotating element 15 provided on the frame 10. The angle change drive device 4 tilts the face board 2 together with the to-be-rotated element 14 by rotating the rotating element 15 by a rotational drive source 17 such as a motor provided on the frame 10. The rotation of the rotational drive source 17 is transmitted to the rotating element 15 via a reduction gear (not shown) such as a worm reduction gear so that the speed of the rotation is reduced. The to-be-rotated element 14 is, for example, formed by attaching a chain 14a in a fixed manner to the outer circumference of a fan-shaped plate member centered on the central axis C1 of the tilting central shaft 12. The rotating element 15 is a sprocket having a claw 15a formed on the outer circumference thereof and to be engaged with the chain 14a. The to-be-rotated element 14 and the rotating element 15 may be gears having teeth engaged with each other.

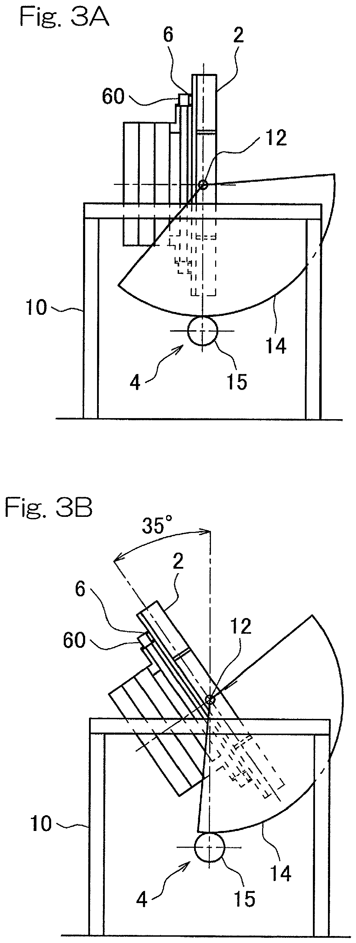

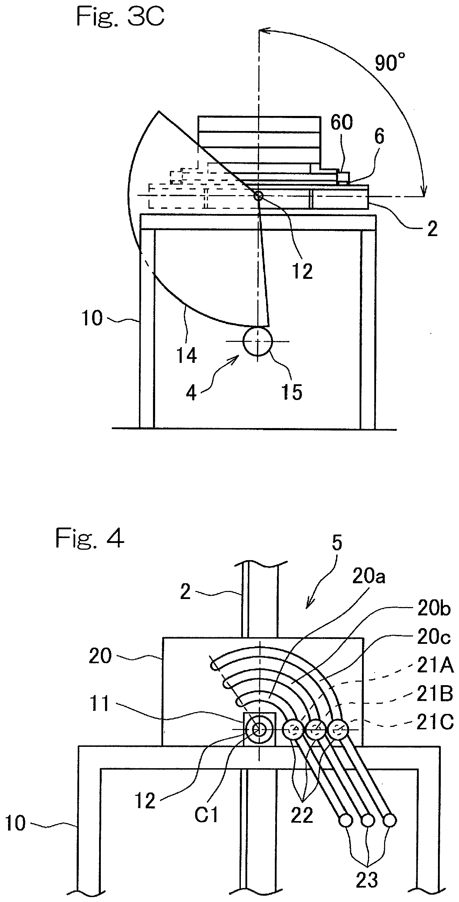

As shown in Figs. 3A to 3C, the orientation of the face board 2 can be changed in a range from a horizontal orientation to a vertical orientation through a tilted orientation by the angle change drive device 4. The angle change drive device 4 is capable of changing the orientation of the face board 2, for example, relative to the vertical orientation in Fig. 3A as a reference, from a forward tilt orientation at 35 degrees in Fig. 3B to a backward tilt orientation at 90 degrees (horizontal) in Fig. 3C. The CT scanner device 70 (Fig. 8) conducts a test while changing the tilt of the rotating mount 71 (Fig. 8) in a range of about ± 30 degrees.

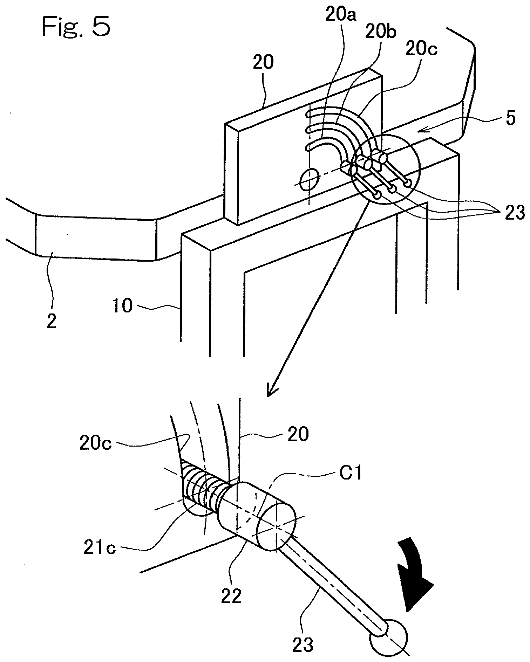

The face board fixing mechanism 5 is a mechanism for fixing the face board 2 to the face board support mechanism 3, at any tilt angle or a predetermined tilt angle. Figs. 4 and 5 show an example of the face board fixing mechanism 5. The face board fixing mechanism 5 has a fixation plate 20 having a plurality of arc-shaped guide grooves 20a, 20b, and 20c which are different from each other in their radiuses and concentric about the central axis C1 of the tilting central shaft 12. The fixation plate 20 is provided on the frame 10. Screw shafts 21A, 21B, and 21C with their ends screwed into a side surface of the face board 2 are inserted through the respective guide grooves 20a, 20b, and 20c of the fixation plate 20. Each screw shaft 21A, 21B, 21C is provided with a contact member 22 formed integrally therewith and having a greater diameter than those of the screw shafts 21A, 21B, 21C. A rotational operation lever 23 is attached to each contact member 22. The rotational operation lever 23 is turned to screw the screw shaft 21A (21B, 21C) into the face board 2 and cause the contact member 22 to strongly contact with the fixation plate 20, thereby fixing the face board 2 to the fixation plate 20 by means of a friction force between the contact member 22 and the fixation plate 20. In this way, the face board 2 is fixed at any tilt angle.

In place of the above configuration, the screw shafts 21A, 21B, and 21C may be provided in a fixed state to the face board 2, and a nut (not shown) screwed to each screw shaft 21A, 21B, 21C may be fastened to fix the face board 2 to the fixation plate 20 by means of a friction force between the nut and the fixation plate 20. In the example in the drawings, three pairs of the guide grooves 20a, 20b, and 20c and the screw shafts 21A, 21B, and 21C are provided, but the number of the pairs is not limited to three. In the example in Fig. 1, the face board fixing mechanism 5 is provided at each of the right side and the left side of the testing device 1, but may be provided at only one of the right and the left sides.

The dedicated frame 6 is a frame for installing the bearing 60 as the test target on the face board 2. The dedicated frame 6 is fitted to the outer circumference of the bearing 60 as the test target, and is attached in a fitted manner to the frame installation hole 2a of the face board 2. Plural types (for example, three types) of such dedicated frames 6 are prepared respectively for a plurality of bearings 60 as the test targets, which have different outer diameters.

Fig. 6 is a broken side view of a part of the testing device using one type of dedicated frame 6 among the plural types of the dedicated frames 6. As shown in Fig. 6, the dedicated frame 6 is a ring-shaped member, and has: an inner circumferential surface 6a to be fitted to the outer circumference of the bearing 60 as the test target; and a receiving surface 6b which protrudes radially inward from one end in the axial direction of the inner circumferential surface 6a and is to receive a side surface of the bearing 60 as the test target. In addition, the dedicated frame 6 has: an outer circumferential surface 6c to be fitted to the frame installation hole 2a of the face board 2; and a flange portion 6d which protrudes from one end in the axial direction of the outer circumferential surface 6c and is to be mounted on the face board 2.

The bearing 60 as the test target is held by the dedicated frame 6 with the outer circumference of the outer ring 60a being fitted to the inner circumferential surface 6a of the dedicated frame 6 and one end surface of the outer ring 60a being received by the receiving surface 6b of the dedicated frame 6. In this condition, a bolt 33 is inserted through a bolt insertion hole 61 formed along the axial direction in the outer ring 60a, and a thread portion of the bolt 33 is screwed into a screw hole 34 formed in the dedicated frame 6, whereby the outer ring 60a is fixed to the dedicated frame 6. A plurality of the bolt insertion holes 61 and a plurality of the screw holes 34 are provided, along respective concentric circles, in the outer ring 60a and the dedicated frame 6, respectively.

Through holes (not shown) may be formed along the axial direction in the face board 2 and the dedicated frame 6, and a screw hole (not shown) may be formed in the outer ring 60a, whereby the outer ring 60a may be fastened to be fixed to the dedicated frame 6 by a bolt (not shown) inserted from the face board 2 side. However, in light of handling performance, attachment performance, and the like, it is desirable that the outer ring 60a is fastened to be fixed to the dedicated frame 6 by the bolt 33 inserted from the outer ring 60a side, as in the present embodiment.

The dedicated frame 6 is held by the face board 2 such that the outer circumferential surface 6c is fitted to the frame installation hole 2a of the face board 2 and the flange portion 6d is mounted on the face board 2. Then, a bolt 37 is inserted through a bolt insertion hole 36 formed along the axial direction in the dedicated frame 6, and a thread portion of the bolt 37 is screwed into a screw hole 38 formed in the face board 2, whereby the dedicated frame 6 is fixed to the face board 2. A plurality of the bolt insertion holes 36 and a plurality of the screw holes 38 are provided, along respective concentric circles, in the dedicated frame 6 and the face board 2, respectively.

In addition, the face board 2 has screw holes 38 on each of a plurality of (in this example, three) concentric circles having different diameters. The screw holes 38 on the respective concentric circles correspond to the bolt insertion holes 36 of the plural types of dedicated frames 6, respectively. Fig. 6 shows a state in which the bolt 37 is screwed into the screw hole 38 provided on the concentric circle having the smallest diameter and thereby the dedicated frame 6 is fixed to the face board 2. The dedicated frame 6 in this case is the one for fixing the bearing 60 as the test target having the smallest outer diameter, among the three types of dedicated frames 6.

Among the three types of dedicated frames 6, the dedicated frame 6 for fixing the bearing 60 as the test target having the middle outer diameter has a sectional shape shown in, for example, Fig. 7A, and by screwing the bolt 37 into the screw hole 38 provided on the concentric circle having the middle diameter, the dedicated frame 6 is fixed to the face board 2. Among the three types of dedicated frames 6, the dedicated frame 6 for fixing the bearing 60 as the test target having the largest outer diameter has a sectional shape shown in, for example, Fig. 7B, and by screwing the bolt 37 into the screw hole 38 provided on the concentric circle having the largest diameter, the dedicated frame 6 is fixed to the face board 2. Although these dedicated frames 6 have different sectional shapes, each of the dedicated frames 6 has the inner circumferential surface 6a, the receiving surface 6b, the outer circumferential surface 6c, and the flange portion 6d.

In Figs. 1 and 2, the bearing rotating mechanism 7 transmits rotation of a bearing rotation motor 52 provided on a bracket 51 fixed to the face board 2, to an inner ring 60b of the bearing 60 as the test target, via a belt transmission device 53. The belt transmission device 53 includes a driving pulley 54 attached to an output shaft 52a of the bearing rotation motor 52, a driven pulley 55 fixed to the inner ring 60b of the bearing 60 as the test target, and a belt 56 wound around the driving pulley 54 and the driven pulley 55. The driven pulley 55 is provided concentrically with the inner ring 60b, and as shown in Fig. 6, is fixed to the inner ring 60b by a bolt 57 through a large-diameter portion 55b which protrudes toward the outer diameter side relative to a belt wound portion 55a. For example, the bearing rotating mechanism 7 is capable of rotating the inner ring 60b of the bearing 60 as the test target at a rotation speed of about 400 revolutions per minute.

On a surface of the driven pulley 55 opposite to a surface thereof that contacts with the inner ring 60b of the bearing 60 as the test target, a plurality of weights 58A, 58B, and 58C for applying a moment load to the inner ring 60b are attached by bolts 59 in a stacked manner. The weight value of each of the weights 58A, 58B, and 58C and the number of the weights to be attached may be arbitrarily selected. Since the driven pulley 55 and the weights 58A, 58B, and 58C are located on the same side in the axial direction with respect to the bearing 60 as the test target, the moment load can be effectively applied to the bearing 60 as the test target.

Sensors (not shown) for detecting torque acting on the bearing 60 as the test target, and vibration, heat, sound, etc. generated during rotation may be attached at appropriate positions in the testing device 1.

A usage method of the testing device 1 will be described.

By the face board support mechanism 3 and the angle change drive device 4, the face board 2 is set in the horizontal orientation as shown in Fig. 3C. The dedicated frame 6 fitted to the outer circumference of the bearing 60 as the test target is attached in a fitted manner to the frame installation hole 2a of the face board 2 set in the horizontal state, whereby the bearing 60 as the test target is installed on the face board 2. The bearing 60 as the test target may be fixed to the dedicated frame 6 after the dedicated frame 6 is fixed to the face board 2, or the dedicated frame 6 may be fixed to the face board 2 after the bearing 60 as the test target is fixed to the dedicated frame 6. In either case, since the face board 2 is in a horizontal orientation, it is only necessary to lower the dedicated frame 6 or the bearing 60 set in a horizontal state, from above onto the face board 2 by using a crane or the like. It is not necessary to support the dedicated frame 6 or the bearing 60 in a proper orientation by people. Therefore, even when the bearing 60 as the test target is the thin-walled large-sized bearing with a great weight, the bearing 60 as the test target can be easily installed on the face board 2. Since the dedicated frame 6 is fitted to the frame installation hole 2a of the face board 2, and the outer circumference of the bearing 60 as the test target is fitted to the inner circumferential surface 6a of the dedicated frame 6, the positions of the dedicated frame 6 and the bearing 60 as the test target are prevented from being displaced relative to the upper surface of the face board 2. Therefore, the bearing 60 as the test target can be accurately installed with the center thereof aligned with the center O of the face board 2.

After the bearing 60 as the test target is installed on the face board 2, the driven pulley 55 and the weights 58A, 58B, and 58C are attached to the inner ring 60b of the bearing 60 as the test target. The driven pulley 55 and the weights 58A, 58B, and 58C may be attached to the inner ring 60b of the bearing 60 as the test target before the dedicated frame 6 is installed on the face board 2. Finally, the belt 56 is wound around the driving pulley 54 and the driven pulley 55, whereby test preparation is completed.

After the test preparation is completed, the face board 2 is set in the vertical orientation or the tilted orientation, to conduct a performance evaluation test of the bearing 60 as the test target. At this time, the face board 2 is fixed at a determined tilt angle by the face board fixing mechanism 5. Thereby, the tilt angle of the face board 2 can be reliably prevented from being unintentionally changed during a test or the like, thus ensuring security. The performance evaluation test is conducted by rotating the inner ring 60b of the bearing 60 as the test target by the bearing rotation motor 52 and then reading a value detected by each sensor. Since the face board 2 can be changed at any tilt angle or a predetermined tile angle, the performance evaluation test of the bearing 60 as the test target can be conducted in a state close to the usage state. In addition, since a moment load is applied to the bearing 60 as the test target by the weights 58A, 58B, and 58C, it is possible to conduct a performance evaluation test while reproducing a state in which an examination device or the like is attached to an object (for example, a rotating mount of a CT scanner) rotatably supported by the bearing 60.

In a normal performance evaluation test, the center of gravity of the weights 58A, 58B, and 58C is positioned coaxially with the center O of the face board 2, so that the test is conducted without an eccentric load being applied to the bearing 60 as the test target. However, this testing device 1 also allows a performance evaluation test in a state in which an eccentric load is applied to the bearing 60 as the test target. This is because the outer circumference of the bearing 60 as the test target is fitted to the inner circumferential surface 6a of the dedicated frame 6 and therefore the eccentric load can be received by the face board 2 via the dedicated frame 6.

As a configuration of the testing device 1 for applying an eccentric load to the bearing 60 as the test target, for example, other weights (not shown) having the center of gravity shifted from the center O of the face board 2 may be used instead of the weights 58A, 58B, and 58C, the weights 58A, 58B, and 58C may be attached to the driven pulley 55 so as to be eccentric in the radial direction, or a weight (not shown) for eccentric load application may be detachably attached to a part in the circumferential direction of the weights 58A, 58B, and 58C. Any of these configurations may be employed.

As an example of a performance evaluation test conducted in a state in which an eccentric load is applied to the bearing 60 as the test target, for example, deflection of the inner ring 60b may be measured while an eccentric load is applied to the bearing 60 as the test target. For measurement of deflection of the inner ring 60b, for example, a dial gauge (not shown) may be used. In this case, even if the weights 58A, 58B, and 58C are provided on the front surface side of the bearing 60 as the test target as in the example shown in the drawings, it is possible to measure deflection of the inner ring 60b by inserting the dial gauge from the back surface side of the face board 2 into the frame installation hole 2a of the face board 2 and an opening portion of the dedicated frame 6 having a ring shape.

The CT scanner device 70 shown in Fig. 8 will be described. The CT scanner device 70 includes: an examination unit 73 having an opening portion 72; and a cradle 74 movable in the opening portion 72. In the examination unit 73, the rotating mount 71 on the inner circumferential side is rotatably supported via two bearings 60 as rolling test targets on a fixation portion 75 on the outer circumferential side. The rotating mount 71 is provided with an X-ray tube 76 and an X-ray detector 77 located opposite to each other in the diameter direction. The cradle 74 on which an examination subject 78 such as a patient is laid is inserted into the opening portion 72 of the examination unit 73, the rotating mount 71 is rotated around the cradle 74 while an X-ray is applied from the X-ray tube 76, and then the X-ray transmitted through the examination subject is detected by the X-ray detector 77, whereby a sectional image of the examination subject 78 is obtained.

### [Reference Numerals]

- 1 ···: Testing device
- 2 ···: Face board
- 3 ···: Face board support mechanism
- 4 ···: Angle change drive device
- 5 ···: Face board fixing mechanism
- 6 ···: Dedicated frame
- 6a ···: Inner circumferential surface
- 6b ···: Receiving surface
- 6c ···: Outer circumferential surface
- 6d ···: Flange portion
- 7 ···: Bearing rotating mechanism
- 12 ···: Tilting central shaft
- 52 ···: Bearing rotation motor
- 58A, 58B, 58C ···: Weight
- 60 ···: Bearing as test target
- 70 ···: CT scanner device
- 71 ···: Rotating mount
- C1 ···: Central axis of tilting central shaft

## Claims

1. A testing device for a thin-walled large-sized bearing, comprising:
a face board configured to allow the thin-walled large-sized bearing as a test target to be installed thereon;
a face board support mechanism configured to support the face board such that the face board can be tilted about a tilting central shaft of the face board aligned with or parallel to a diameter line of the bearing installed on the face board, with the tilting central shaft being horizontally supported;
an angle change drive device configured to change a tilt angle of the face board;
a bearing rotation motor installed on the face board and configured to rotate an inner ring of the bearing; and
a weight detachably attached to the inner ring of the bearing, to generate a moment load to the bearing,
wherein the face board support mechanism and the angle change drive device cooperatively support the face board so as to allow change in an orientation of the face board in a range from a horizontal orientation to a vertical orientation through a tilted orientation, and
the face board is provided with a dedicated frame fitted to an outer circumference of the bearing and attached in a fitted manner to a frame installation hole formed in the face board.

2. The testing device for the thin-walled large-sized bearing, as claimed in claim 1, wherein
the dedicated frame has: an inner circumferential surface to be fitted to the outer circumference of the bearing; a receiving surface protruding radially inward from one end in an axial direction of the inner circumferential surface to receive a side surface of the bearing; an outer circumferential surface to be fitted to the frame installation hole of the face board; and a flange portion protruding from one end in the axial direction of the outer circumferential surface and is mounted on the face board.

3. The testing device for the thin-walled large-sized bearing, as claimed in claim 1 or 2, further comprising, as the dedicated frame, plural types of dedicated frames to be installed in an exchangeable manner to the face board, the plural types of dedicated frames respectively allowing bearings having different outer diameters to be fitted thereto.

4. The testing device for the thin-walled large-sized bearing, as claimed in any one of claims 1 to 3, further comprising a face board fixing mechanism provided separately from the angle change drive device and configured to fix the face board to the face board support mechanism at any tilt angle or a predetermined tilt angle.

5. The testing device for the thin-walled large-sized bearing, as claimed in any one of claims 1 to 4, wherein the bearing is a bearing to support a rotating mount of a CT scanner device.
